# EUROPÄISCHE PATENTSCHRIFT

(11) **EP 3 464 533 B1**
(45) Veröffentlichungstag und Bekanntmachung des Hinweises auf die Patenterteilung: **21.10.2020**
(21) Anmeldenummer: 17722045.6
(22) Anmeldetag: 11.05.2017
(51) Int. Cl.: A61Q 19/10, A61Q 9/02, A61Q 11/00, A61Q 5/02, A61K 8/36, A61K 8/41, A61K 8/46, C11D 1/12, C11D 1/28, C11D 1/83, C11D 1/90, C11D 1/94, C11D 3/04

(54) **WÄSSRIGE TENSID-ZUSAMMENSETZUNGEN**
AQUEOUS TENSIDE COMPOSITIONS
COMPOSITIONS AQUEUSES DE TENSIOACTIFS

(30) Priorität: 07.06.2016 EP 16173321
(43) Veröffentlichungstag der Anmeldung: 10.04.2019
(73) Patentinhaber: BASF SE, 67056 Ludwigshafen am Rhein (DE)
(72) Erfinder: BRUNN, Claudia, 40589 Düsseldorf-Holthausen (DE); BEHLER, Ansgar, 40589 Düsseldorf-Holthausen (DE)
(74) Vertreter: BASF IP Association
(86) Internationale Anmeldenummer: PCT/EP2017/061314
(87) Internationale Veröffentlichungsnummer: WO 2017/211535

(56) Entgegenhaltungen:
- WO-A1-98/44907
- WO-A1-2015/117842
- DE-A1- 3 707 014
- DE-A1- 4 220 580

## Beschreibung

### Gebiet der Erfindung

Die vorliegende Erfindung betrifft wässrige Tensid-Zusammensetzungen mit einem Gehalt an alpha-Sulfofettsäuredisalzen, Sulfoketonen, Seifen, anorganische Salze der Schwefelsäure und speziellen Betainen (X).

### Stand der Technik

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzt auch Ethersulfate). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaum- oder Duschbädern, aber auch in Handgeschirrspülmitteln.

Für viele aktuelle Anwendungen werden an anionische Tenside außer einer guten grenzflächenaktiven Wirkung weitere Anforderungen gestellt. Insbesondere in der Kosmetik ist eine hohe dermatologische Verträglichkeit erforderlich. Des Weiteren ist in der Regel ein gutes Schaumvermögen und eine angenehme Sensorik des Schaumes erwünscht. Des Weiteren besteht ein Bedarf an anionischen Tensiden, die zumindest teilweise aus biogenen Quellen und speziell auch nachwachsenden Rohstoffen hergestellt werden können.

WO-A-92/15660 offenbart flüssige Reinigungsmittel mit einem Gehalt an sulfoölsauren Disalzen. Es wird offenbart, dass sulfoölsauren Disalze in der Lage sind, die Viskosität von Tensiden oder Tensidgemischen für Reinigungsmittel - insbesondere auf Basis von Fettalkylsulfaten, Fettalkylethersulfaten, Alkylpolyglucosiden und Fettsäuremonoethanolamiden - zu senken und zwar ebenso effektiv oder sogar besser wie durch Zusatz von Ethanol oder Hydrotropen (Seite 2, zweiter Absatz).

WO-A-2011/049,932 offenbart wässrige Zusammensetzungen enthaltend a) wenigstens ein Hydrotrop, b) wenigstens ein Alkylbetain oder Alkylsultain, 0 bis etwa 3% Elektrolyt. Bei den Hydrotropen a) kann es sich um sulfonierte Fettsäuren oder deren Ester handeln, die in saurer Form oder als Salz und auch in Mischungen untereinander vorliegen können.

WO-A-2015/117842 offenbart wässrige Tensid-Zusammensetzungen enthaltend ein oder mehrere alpha-Sulfofettsäuredisalze, ein oder mehrere Amidoalkylbetaine, Wasser und gegebenenfalls Seifen und anorganische Salze der Schwefelsäure. Diese Zusammensetzungen sind transparent, lagerstabil, zeichnen sich durch gutes Schaumvermögen und gute Viskosität aus und eignen sich für kosmetische Mittel sowie Wasch- und Reinigungsmittel.

DE-A-42 20 580 offenbart Sulfocarbonylverbindungen sowie ihre Verwendung zur Herstellung oberflächenaktiver Mittel.

### Beschreibung der Erfindung

Die Aufgabe der vorliegenden Erfindung hat darin bestanden, wässrige Tensid-Zusammensetzungen bereitzustellen, die sich durch die im Folgenden genannten Eigenschaften auszeichnen:
- Gutes Schaumvermögen.
- Angenehme Sensorik des Schaumes.
- Gute Hautverträglichkeit.

Gegenstand der Erfindung sind zunächst wässrige Tensid-Zusammensetzungen enthaltend
- ein oder mehrere **alpha-Sulfofettsäuredisalze (A)** der allgemeinen Formel (I),

   R¹CH(SO₃M¹)COOM² (I),

   worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
- ein oder mehrere **Sulfoketone (B)**, die ausgewählt sind aus den Verbindungen (F) und den Verbindungen (G),
   wobei die Verbindungen (F) die allgemeine Formel (VI)

   R⁶CH₂-CO-CHR⁷(SO₃M⁸) (VI),

   aufweisen, worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine,
   und wobei die Verbindungen (G) die allgemeine Formel (VII)

   (SO₃M⁹)R⁸CH-CO-CHR⁹(SO₃M¹⁰) (VII),

   aufweisen, worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine,
- ein oder mehrere **Betaine (X)** der allgemeinen Formel (II)

   R⁷³-N⁺(CH₃)₂-CH₂COO⁻ (II)

   wobei der Rest R⁷³ einen linearen oder verzweigten Alkylrest mit 8 bis 20 C-Atomen bedeutet,
- ein oder mehrere **Verbindungen (C)** der allgemeinen Formel (III)

   R⁴COOM⁵ (III)

   wobei der Rest R⁴ einen linearen oder verzweigten Alkyl- oder Alkenylrest mit 7 bis 19 C-Atomen bedeutet und der Reste M⁵ wird ausgewählt aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin,
- ein oder mehrere **anorganische Salze der Schwefelsäure (D)** der allgemeinen Formel (IV)

   (M⁶)₂SO₄ (IV)

   wobei M⁶ ausgewählt wird aus der Gruppe Li, Na, K, Ca/2, Mg/2, Ammonium und Alkanolamin. Besonders bevorzugte Alkanolamine sind dabei Monoethanolamin, Diethanolamin, Triethanolamin und Mono-Isopropanolamin,
- **Wasser**,
   wobei folgende Maßgabe gilt:
- sofern die wässrigen Tensid-Zusammensetzungen ein oder mehrere Estersulfonate (E) der allgemeinen Formel (V),

   R²CH(SO₃M⁷)COOR³ (V)

   worin der Rest R² einen linearen oder verzweigten Alkyl- oder Alkenylrest mit 6 bis 18 C-Atomen bedeutet und der Rest R³ einen linearen oder verzweigten Alkyl- oder Alkenylrest mit 1 bis 20 C-Atomen bedeutet, wobei der Rest R³ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E)-zu 90 Gew.-% oder mehr - vorliegen müssen.

Die erfindungsgemäßen wässrigen Tensid-Zusammensetzungen zeichnen sich durch folgende vorteilhaften Eigenschaften aus:
- Gutes Schaumvermögen und angenehme Sensorik des Schaumes. Hierzu sei angemerkt, dass insbesondere im Bereich der Kosmetik unter Schaumvermögen verschiedene Aspekte verstanden werden können, beispielsweise können sowohl Schaumvolumen, Schaumstabilität, Schaumelastizität, Wassergehalt des Schaumes als auch optische Merkmale des Schaumes wie beispielsweise die Porengröße zur Beurteilung des Schaumes herangezogen werden. Die erfindungsgemäßen Zusammensetzungen weisen ein großes Schaumvolumen während des Anschäumens auf. In der Praxis findet das Anschäumen in einem relativ kurzen Zeitraum statt (von wenigen Sekunden bis zu einer Minute). Typischerweise wird beim Anschäumen ein Duschgel oder ein Shampoo durch Reiben zwischen Händen, Haut und/oder Haaren verteilt und zum Schäumen gebracht. Im Labor kann das Anschäumverhalten einer wässrigen Tensidlösung z.B. dadurch beurteilt werden, dass man in einer vergleichbar kurzen Zeitspanne die Lösung durch Rühren, Schütteln, Pumpen, Durchperlen eines Gasstroms oder auf andere Weise in Bewegung versetzt. Eine subjektive Beurteilung der Schaumsensorik kann in einem Probandentest erfolgen. Hierbei können Aspekte wie beispielsweise Cremigkeit, Elastizität, Formbarkeit des Schaums beurteilt werden.
- Gute Haut- bzw. Schleimhaut-Verträglichkeit. Diese kann durch den Fachmann bekannte invitro Methode (bspw. RBC oder HET-CAM) als auch Probandentests (bspw. Patchtest) nachgewiesen werden.
- Hervorragende Pflegeleistung an Haut und Haar. Diese kann beispielsweise im Probandentest anhand des subjektiven Hautgefühls (Glätte, Trockenheit) oder Haptik und Griff des behandelten Haares beurteilt werden. Es können ebenso mechanische Messmethoden wie bspw. Kämmarbeit am Haar herangezogen werden.
- Gute Lagerstabilität. Diese ist dann gegeben, wenn die wässrigen Zusammensetzungen über einen Zeitraum von mehreren Wochen keine sichtbaren (z.B. Austrübung, Verfärbung, Phasentrennung) oder messbaren (z.B. pH-Wert, Viskosität, Aktivsubstanzgehalt) Veränderungen erfahren.
- Gute Anwendbarkeit und Verarbeitbarkeit. Die Zusammensetzungen lassen sich bei Einbringen in Wasser schnell und ohne Wärmezufuhr verlösen.
- Gute Klarlöslichkeit und Transparenz. Die wässrigen Tensid-Zusammensetzungen neigen nicht zu Ausfällungen oder Austrübungen.
- Ausreichend hohe Viskosität, worunter im Rahmen der vorliegenden Erfindung ein Wert von 1000 mPas oder höher verstanden wird (gemessen mit einem Brookfield RV Laborrheometer bei 23°C, 12 U/min, Spindelset RV 02 bis 07 (Spindelauswahl je nach Viskositätbereich)). "mPas" bedeutet bekanntlich Millipascalsekunden
- Gute Reinigungsleistung. Die wässrigen Tensidzusammensetzungn eignen sich, um Anschmutzungen, insbesondere fett- oder ölhaltige Verschmutzungen, von festen oder textilen Oberflächen zu entfernen und zu emulgieren.

### Zu den Verbindungen (A)

Die Verbindungen (A), die im Rahmen der vorliegenden Erfindung als **alpha-Sulfofettsäuredisalze** bezeichnet werden, sind für die erfindungsgemäßen wässrigen Tensid-Zusammensetzungen obligatorisch. Sie haben die oben angegebene Formel (I)

R¹CH(SO₃M¹)COOM² (I),

worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine. Dabei gelten für die Verbindungen (A) die oben genannten Bedingungen:

In einer Ausführungsform beträgt der Anteil der Verbindungen (A), bei denen der Rest R¹ ein Alkenylrest ist, - bezogen auf die Gesamtmenge der Verbindungen (A) in den wäßrigen Tensid-Zusammensetzungen -bei 3 Gew.-% oder weniger.

Bezüglich der Reste M¹ und M² besonders bevorzugte Alkanolamine werden dabei ausgewählt aus der Gruppe Monoethanolamin, Diethanolamin, Triethanolamin und Mono-Isopropanolamin.

In einer bevorzugten Ausführungsform bedeutet der Rest R¹ in der Formel (I) einen gesättigten, linearen Alkylrest mit 10 bis 16 C-Atomen, wobei in Bezug auf die Verbindungen (A) gilt, dass der Anteil der Verbindungen (A), bei denen der Rest R¹ ein Decyl- und/oder ein Dodecylrest ist, - bezogen auf die Gesamtmenge der Verbindungen (A) - bei 70 Gew.-% oder mehr und insbesondere bei 90 Gew.-% oder mehr liegt.

Vorzugsweise werden die Reste M¹ und M² in der Formel (I) ausgewählt aus der Gruppe H (Wasserstoff) und Na (Natrium).

Die Verbindungen (A) können nach allen dem Fachmann einschlägig bekannten Methoden hergestellt werden. Eine besonders bevorzugte Methode der Herstellung ist dabei die Sulfierung der entsprechenden Carbonsäuren. Dabei setzt man die entsprechenden Carbonsäure und insbesondere die entsprechenden Fettsäuren mit gasförmigem Schwefeltrioxid um, wobei man das Schwefeltrioxid vorzugsweise in einer Menge einsetzt, dass das molare Verhältnis von SO₃ zu Fettsäure im Bereich von 1,0 : 1 bis 1,1 : 1 liegt. Die so erhaltenen Rohprodukte, die saure Sulfierprodukte darstellen, werden anschließend partiell oder vollständig neutralisiert, wobei eine vollständige Neutralisation mit wässriger NaOH bevorzugt ist. Gewünschtenfalls können auch Reinigungsschritte und/oder eine Bleiche (zur Einstellung der gewünschten hellen Farbe der Produkte) vorgenommen werden.

In einer besonders bevorzugten Ausführungsform werden die Verbindungen (A) in technischer Form eingesetzt. Dies bedeutet, dass man die entsprechenden Carbonsäuren, insbesondere native Fettsäure, mit gasförmigem Schwefeltrioxid sulfiert, wodurch nach partieller oder vollständiger Neutralisation der entstehenden sauren Sulfierprodukte ein Gemisch der Verbindungen (A), (C) und (D) resultiert. Durch entsprechende Einstellungen der Reaktionsparameter (insbesondere Mol-Verhältnis von Carbonsäure und Schwefeltrioxid sowie Reaktionstemperatur) lässt sich das Verhältnis der Verbindungen (A), (C) und (D) steuern. Die Verbindungen (C) und (D) sind unten beschrieben.

Im Rahmen der vorliegenden Erfindung sind solche technischen Mischungen der alpha-Sulfofettsäuredisalze bevorzugt, die wie folgt zusammengesetzt sind:
- Der Gehalt an (A) liegt im Bereich von 60 bis 100 Gew.-%,
- der Gehalt an (C) liegt im Bereich von 0 bis 20 Gew.-%,
- der Gehalt an (D) liegt im Bereich von 0 bis 20 Gew.-%,
mit der Maßgabe, dass die Summe der Komponenten (A), (C) und (D) in dieser Mischung 100 Gew.-% beträgt.

### Zu den Verbindungen (B)

Wie oben ausgeführt enthalten die erfindungsgemäßen wässrigen Tensid-Zusammensetzungen neben den Verbindungen (A) und Wasser ein oder mehrere **Sulfoketone (B)**, die ausgewählt werden aus den Verbindungen (F) und (G).

Die **Verbindungen (F)** haben die allgemeine Formel (VI)

R⁶CH₂-CO-CHR⁷(SO₃M⁸) (VI),

worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Die Verbindungen (F) werden im Rahmen der vorliegenden Erfindung als Mono-Sulfo-Ketone bezeichnet.

In einer bevorzugten Ausführungsform bedeuten die Reste R⁶ und R⁷ in der Formel (VI) - unabhängig voneinander - einen gesättigten, linearen Rest mit 10 bis 16 C-Atomen, wobei in Bezug auf die Verbindungen (F) gilt, dass der Anteil der Verbindungen (F), bei denen die Reste R⁶ und R⁷ einen Decyl- und/oder ein Dodecylrest bedeuten, - bezogen auf die Gesamtmenge der Verbindungen (F) - bei 70 Gew.-% oder mehr und vorzugsweise bei 90 Gew.-% oder mehr liegt. In einer Ausführungsform wird der Rest M⁸ in der Formel (VI) ausgewählt aus der Gruppe H und Na.

Die **Verbindungen (G)** haben die allgemeine Formel (VII)

(SO₃M⁹)R⁸CH-CO-CHR⁹(SO₃M¹⁰) (VII),

worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Die Verbindungen (G) werden im Rahmen der vorliegenden Erfindung als Di-Sulfo-Ketone bezeichnet.

In einer bevorzugten Ausführungsform bedeuten die Reste R⁸ und R⁹ in der Formel (VII) - unabhängig voneinander - einen gesättigten, linearen Rest mit 10 bis 16 C-Atomen, wobei in Bezug auf die Verbindungen (G) gilt, dass der Anteil der Verbindungen (G), bei denen die Reste R⁸ und R⁹ einen Decyl- und/oder ein Dodecylrest bedeuten, - bezogen auf die Gesamtmenge der Verbindungen (G) - bei 70 Gew.-% oder mehr und vorzugsweise bei 90 Gew.-% oder mehr liegt. In einer Ausführungsform werden die Reste M⁹ und M¹⁰ in der Formel (VII) ausgewählt aus der Gruppe H und Na.

Die Herstellung der Verbindungen (F) und (G) unterliegt keinen besonderen Einschränkungen und sie können nach allen dem Fachmann bekannten Methoden hergestellt werden.

In einer Ausführungsform werden die Verbindung (F) und (G) durch Sulfonierung entsprechender Ketone mit gasförmigem Schwefeltrioxid hergestellt, wie in der deutschen Offenlegungsschrift DE-A-42,20,580 beschrieben.

In einer anderen Ausführungsform geht man zur Herstellung der Verbindungen (F) und (G) von Fettsäuren aus. Dabei führt man die Sulfierung von flüssigen Fettsäuren mit gasförmigem Schwefeltrioxid so durch, dass dabei neben Disalzen (A) auch die Verbindungen (F) und (G) entstehen, was dadurch realisiert werden kann, dass man die Sulfierung wie folgt durchführt: Das Verhältnis der Rohstoffe Fettsäure, die auch in Form von Gemischen von Fettsäuren unterschiedlicher Kettenlänge eingesetzt werden können, und Schwefeltrioxid wird so eingestellt, dass man 1,0 bis 1,5 mol und insbesondere 1,0 bis 1,25 mol SO₃ pro mol Fettsäure(n) einsetzt. Die Fettsäuren werden dabei mit einer Vorlagetemperatur im Bereich von 70 bis 100 °C in den Reaktor eingebracht. Nach der Sulfierung wird das erhaltene flüssige Sulfierprodukt in einer temperierten Nachreaktionsschlange für 5 bis 20 Minuten auf dieser Temperatur gehalten und gealtert. Anschließend erfolgt die Neutralisation mit einer wässrigen Base, vorzugsweise Natriumhydroxid, in der Regel bei einem pH-Wert im Bereich von von 5 bis 10, insbesondere von 5 bis 7. Im Anschluss kann eine saure Bleiche - der pH wird hierbei auf einen Wert von 7 oder weniger eingestellt - mit Wasserstoffperoxid durchgeführt werden.

### Zu den Verbindungen (X)

Die Verbindungen (X), die im Rahmen der vorliegenden Erfindung als **Betaine** bezeichnet werden, sind für die erfindungsgemäßen wässrigen Tensidzusammensetzungen obligatorisch. Sie haben die allgemeine Formel (II)

R⁷³-N⁺(CH₃)₂-CH₂COO⁻ (II)

wobei der Rest R⁷³ einen linearen oder verzweigten Alkylrest mit 8 bis 20 C-Atomen bedeutet. Die Verbindungen (X) können nach allen dem Fachmann einschlägig bekannten Methoden hergestellt werden, beispielsweise durch Reaktion tertiärer Amine mit Chloressigsäure in Gegenwart von Natriumhydroxid.

In einer bevorzugten Ausführungsform bedeutet der Rest R⁷³ in der Formel (IIa) einen linearen Alkylrest mit 12 bis 18 C-Atomen.

Vorzugsweise handelt es sich bei dem Betain um ein Coco Betaine (INCI-Bezeichnung).

### Zu den Verbindungen (C)

Die Verbindungen (C) sind für die erfindungsgemäßen wässrigen Tensidzusammensetzungen obligatorisch. Die Verbindungen (C) haben die allgemeine Formel (III)

R⁴COOM⁵ (III)

In der Formel (III) bedeutet der Rest R⁴ einen linearen oder verzweigten Alkyl- oder Alkenylrest mit 7 bis 19 C-Atomen und der Rest M⁵ wird ausgewählt aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

### Zu den Verbindungen (D)

Die Verbindungen (D), die im Rahmen der vorliegenden Erfindung als anorganische Salze der Schwefelsäure (D) bezeichnet werden, sind für die erfindungsgemäßen wässrigen Tensidzusammensetzungen obligatorisch. Die Verbindungen (D) haben die allgemeine Formel (IV)

(M⁶)₂SO₄ (IV)

wobei M⁶ ausgewählt wird aus der Gruppe Li, Na, K, Ca/2, Mg/2, Ammonium und Alkanolamin. Besonders bevorzugte Alkanolamine sind dabei Monoethanolamin, Diethanolamin, Triethanolamin und Mono-Isopropanolamin.

Gewünschtenfalls können die erfindungsgemäßen wässrigen Tensid-Zusammensetzungen zusätzlich ein oder mehrere weitere Tenside enthalten, die strukturell nicht zu den oben genannten Verbindungen (A), (B), (X), (C) oder (D) zählen. Bei diesen Tensiden kann es sich um anionische, kationische, nichtionische oder amphotere Tenside handeln.

### Verwendung der Zusammensetzungen

Ein weiterer Erfindungsgegenstand ist die Verwendung der oben genannten Zusammensetzungen für kosmetische Mittel, sowie Wasch- und Reinigungsmittel.

Im Hinblick auf kosmetische Mittel sind dabei insbesondere solche besonders bevorzugt, die in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten (etwa Zahnpasten, Mundwässern und dergleichen) vorliegen.

Im Hinblick auf Reinigungsmittel sind dabei insbesondere Mittel mit niedrigem pH-Wert zur Reinigung harter Oberflächen bevorzugt, wie Bad- und WC-Reiniger und dergleichen, sowie für Reinigungs- und/oder Duftgele zur Anwendung in sanitären Einrichtungen.

## Patentansprüche

1. Wässrige Tensid-Zusammensetzungen enthaltend
• ein oder mehrere **alpha-Sulfofettsäuredisalze (A)** der allgemeinen Formel (I),
R¹CH(SO₃M¹)COOM² (I),
worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
• ein oder mehrere **Sulfoketone (B)**, die ausgewählt sind aus den Verbindungen (F) und den Verbindungen (G),
wobei die Verbindungen (F) die allgemeine Formel (VI)
R⁶CH₂-CO-CHR⁷(SO₃M⁸) (VI),
aufweisen, worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine,
und wobei die Verbindungen (G) die allgemeine Formel (VII)
(SO₃M⁹)R⁸CH-CO-CHR⁹(SO₃M¹⁰) (VII),
aufweisen, worin die Reste R⁸ und R⁹ - unabhängig voneinander - einen linearen oder verzweigten Alkylrest mit 6 bis 18 C-Atomen bedeuten und die Reste M⁹ und M¹⁰-unabhängig voneinander - ausgewählt werden aus der Gruppe H, Li, Na, K, Ca/2, Mg/2, Ammonium und Alkanolamine,
• ein oder mehrere **Betaine (X)** der allgemeinen Formel (II)
R⁷³-N⁺(CH₃)₂-CH₂COO⁻ (II)
wobei der Rest R⁷³ einen linearen oder verzweigten Alkylrest mit 8 bis 20 C-Atomen bedeutet,
• ein oder mehrere **Verbindungen (C)** der allgemeinen Formel (III)
R⁴COOM⁵ (III)
wobei der Rest R⁴ einen linearen oder verzweigten Alkyl- oder Alkenylrest mit 7 bis 19 C-Atomen bedeutet und der Rest M⁵ wird ausgewählt aus der Gruppe H, Li, Na, K, Ca/2, Mg/2, Ammonium und Alkanolamine, bevorzugte Alkanolamine sind dabei Monoethanolamin, Diethanolamin, Triethanolamin und Mono-Isopropanolamin,
• ein oder mehrere anorganische Salze der Schwefelsäure (D) der allgemeinen Formel (IV)
(M⁶)₂SO₄ (IV)
wobei M⁶ ausgewählt wird aus der Gruppe Li, Na, K, Ca/2, Mg/2, Ammonium und Alkanolamine, bevorzugte Alkanolamine sind dabei Monoethanolamin, Diethanolamin, Triethanolamin und Mono-Isopropanolamin,
• Wasser,
wobei folgende Maßgabe gilt:
• sofern die wässrigen Tensid-Zusammensetzungen ein oder mehrere Estersulfonate (E) der allgemeinen Formel (V),
R²CH(SO₃M⁷)COOR³ (V)
worin der Rest R² einen linearen oder verzweigten Alkyl- oder Alkenylrest mit 6 bis 18 C-Atomen bedeutet und der Rest R³ einen linearen oder verzweigten Alkyl- oder Alkenylrest mit 1 bis 20 C-Atomen bedeutet, wobei der Rest R³ logischerweise erst ab 3 C-Atomen ein Alkenylrest sein der verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) bezogen auf die Gesamtheit der Verbindungen (A) und (E) zu 90 Gew.-% oder mehr vorliegen müssen.

2. Zusammensetzungen nach Anspruch 1, wobei der Rest R¹ in der Formel (I) einen gesättigten, linearen Alkylrest mit 10 bis 16 C-Atomen bedeutet, wobei in Bezug auf die Verbindungen (A) gilt, dass der Anteil der Verbindungen (A), bei denen der Rest R¹ ein Decyl- oder ein Dodecylrest ist, - bezogen auf die Gesamtmenge der Verbindungen (A) - bei 90 Gew.-% oder mehr liegt.

3. Zusammensetzungen nach Anspruch 1 oder 2, wobei die Reste M¹ und M² ausgewählt werden aus der Gruppe H (Wasserstoff) und Na (Natrium).

4. Verwendung der Zusammensetzungen nach einem der Ansprüche 1 bis 3 für kosmetische Mittel sowie Wasch- und Reinigungsmittel.

5. Verwendung der Zusammensetzungen nach Anspruch 4 für kosmetische Mittel in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten.

6. Verwendung der Zusammensetzungen nach Anspruch 4 für Mittel mit niedrigem pH-Wert zur Reinigung harter Oberflächen, wie Bad- und WC-Reiniger und dergleichen, sowie für Reinigungs- und/oder Duftgele zur Anwendung in sanitären Einrichtungen.

## Claims

1. An aqueous surfactant composition comprising
• one or more **alpha-sulfo fatty acid disalts (A)** of general formula (I),
R¹CH(SO₃M¹)COOM² (I),
in which the radical R¹ is a linear or branched alkyl or alkenyl radical having 6 to 18 carbon atoms and the radicals M¹ and M² - independently of one another - are selected from the group comprising H, Li, Na, K, Ca/2, Mg/2, ammonium and alkanolamines,
• one or more **sulfoketones (B)** selected from the compounds (F) and the compounds (G),
in which the compounds (F) have general formula (VI)
R⁶CH₂-CO-CHR⁷(SO₃M⁸) (VI),
in which the radicals R⁶ and R⁷ - independently of one another - are a linear or branched alkyl radical having 6 to 18 carbon atoms and the radical M⁸ is selected from the group comprising H, Li, Na, K, Ca/2, Mg/2, ammonium and alkanolamines,
and wherein the compounds (G) have general formula (VII)
(SO₃M⁹)R⁸CH-CO-CHR⁹(SO₃M¹⁰) (VII),
in which the radicals R⁸ and R⁹ - independently of one another - are a linear or branched alkyl radical having 6 to 18 carbon atoms and the radicals M⁹ and M¹⁰ - independently of one another - are selected from the group comprising H, Li, Na, K, Ca/2, Mg/2, ammonium and alkanolamines,
• one or more **betaines (X)** of general formula (II)
R⁷³-N⁺(CH₃)₂-CH₂COO⁻ (II)
wherein the radical R⁷³ is a linear or branched alkyl radical having 8 to 20 carbon atoms,
• one or more **compounds (C)** of general formula (III)
R⁴COOM⁵ (III)
wherein the radical R⁴ is a linear or branched alkyl or alkenyl radical having 7 to 19 carbon atoms and the radical M⁵ is selected from the group comprising H, Li, Na, K, Ca/2, Mg/2, ammonium and alkanolamines, preferred alkanolamines in this connection are monoethanolamine, diethanolamine, triethanolamine and monoisopropanolamine,
• one or more inorganic salts of sulfuric acid (D) of general formula (IV)
(M⁶)₂SO₄ (IV)
wherein M⁶ is selected from the group comprising Li, Na, K, Ca/2, Mg/2, ammonium and alkanolamines, preferred alkanolamines in this connection are monoethanolamine, diethanolamine, triethanolamine and monoisopropanolamine,
• water,
wherein the following proviso applies:
• if the aqueous surfactant composition comprises one or more ester sulfonates (E) of general formula (V),
R²CH(SO₃M⁷)COOR³ (V)
in which the radical R² is a linear or branched alkyl or alkenyl radical having 6 to 18 carbon atoms and the radical R³ is a linear or branched alkyl or alkenyl radical having 1 to 20 carbon atoms, where the radical R³ is logically an alkenyl radical which can be branched only above 3 carbon atoms, and the radical M⁷ is selected from the group comprising Li, Na, K, Ca/2, Mg/2, ammonium and alkanolamines, it is the case that the compounds (A) - based on the totality of the compounds (A) and (E) - must be present to an extent of 90% by weight or more.

2. The composition according to claim 1, wherein the radical R¹ in the formula (I) is a saturated, linear alkyl radical having 10 to 16 carbon atoms, where with regard to the compounds (A) it is the case that the fraction of the compounds (A) in which the radical R¹ is a decyl or a dodecyl radical - based on the total amount of the compounds (A) - is 90% by weight or more.

3. The composition according to claim 1 or 2, wherein the radicals M¹ and M² are selected from the group comprising H (hydrogen) and Na (sodium).

4. The use of the composition according to any one of claims 1 to 3 for cosmetic products and also detergents and cleaners.

5. The use of the composition according to claim 4 for cosmetic products in the form of hair shampoos, shower gels, soaps, syndets, washing pastes, washing lotions, scrub preparations, foam baths, oil baths, shower baths, shaving foams, shaving lotions, shaving creams and dental care products.

6. The use of the composition according to claim 4 for products with a low pH for cleaning hard surfaces, such as bath and toilet cleaners and the like, and also for cleaning and/or fragrance gels for use in sanitary installations.

## Revendications

1. Compositions aqueuses de tensioactifs, contenant :
- un ou plusieurs **disels d'acides alpha-sulfo-gras (A)** de la formule générale (I),
R¹CH(SO₃M¹)COOM² (I),
dans laquelle le radical R¹ signifie un radical alkyle ou alcényle linéaire ou ramifié de 6 à 18 atomes C, et les radicaux M¹ et M² sont choisis, indépendamment les uns des autres, dans le groupe constitué par H, Li, Na, K, Ca/2, Mg/2, ammonium et alcanolamines,
- une ou plusieurs **sulfocétones (B)**, qui sont choisies parmi les composés (F) et les composés (G), les composés (F) présentant la formule générale (VI) :
R⁶CH₂-CO-CHR⁷(SO₃M⁸) (VI)
dans laquelle les radicaux R⁶ et R⁷ signifient, indépendamment l'un de l'autre, un radical alkyle linéaire ou ramifié de 6 à 18 atomes C, et le radical M⁸ est choisi dans le groupe constitué par H, Li, Na, K, Ca/2, Mg/2, ammonium et alcanolamines,
et les composés (G) présentant la formule générale (VII) :
(SO₃M⁹)R⁸CH-CO-CHR⁹(SO₃M¹⁰) (VII)
dans laquelle les radicaux R⁸ et R⁹ signifient, indépendamment l'un de l'autre, un radical alkyle linéaire ou ramifié de 6 à 18 atomes C, et les radicaux M⁹ et M¹⁰ sont choisis, indépendamment l'un de l'autre, dans le groupe constitué par H, Li, Na, K, Ca/2, Mg/2, ammonium et alcanolamines,
- une ou plusieurs **bétaïnes (X)** de la formule générale (II) :
R⁷³-N⁺(CH₃)₂-CH₂COO- (II)
dans laquelle le radical R⁷³ signifie un radical alkyle linéaire ou ramifié de 8 à 20 atomes C,
- un ou plusieurs **composés (C)** de la formule générale (III) :
R⁴COOM⁵ (III)
dans laquelle le radical R⁴ signifie un radical alkyle ou alcényle linéaire ou ramifié de 7 à 19 atomes C, et le radical M⁵ est choisi dans le groupe constitué par H, Li, Na, K, Ca/2, Mg/2, ammonium et alcanolamines, des alcanolamines préférées étant ici la monoéthanolamine,
la diéthanolamine, la triéthanolamine et la monoisopropanolamine,
- un ou plusieurs sels inorganiques de l'acide sulfurique (D) de la formule générale (IV) :
(M⁶)₂SO₄ (IV)
dans laquelle M⁶ est choisi dans le groupe constitué par Li, Na, K, Ca/2, Mg/2, ammonium et alcanolamines, des alcanolamines préférées étant ici la monoéthanolamine,
la diéthanolamine, la triéthanolamine et la monoisopropanolamine,
- de l'eau,
la condition suivante s'appliquant :
- si les compositions aqueuses de tensioactifs contiennent un ou plusieurs ester-sulfonates (E) de la formule générale (V),
R²CH(SO₃M⁷)COOR³ (V)
dans laquelle le radical R² signifie un radical alkyle ou alcényle linéaire ou ramifié de 6 à 18 atomes C, et le radical R³ signifie un radical alkyle ou alcényle linéaire ou ramifié de 1 à 20 atomes C, le radical R³ ne pouvant logiquement qu'à partir de 3 atomes C être un radical alcényle qui peut être ramifié, et le radical M⁷ est choisi dans le groupe constitué par Li, Na, K, Ca/2, Mg/2, ammonium et alcanolamines, alors les composés (A) doivent être présents à hauteur de 90 % en poids ou plus par rapport à la totalité des composés (A) et (E).

2. Compositions selon la revendication 1, dans lesquelles le radical R¹ dans la formule (I) signifie un radical alkyle linéaire, saturé, de 10 à 16 atomes C, au regard des composés (A), la proportion de composés (A) dans lesquels le radical R¹ est un radical décyle ou dodécyle, par rapport à la quantité totale de composés (A), étant de 90 % en poids ou plus.

3. Compositions selon la revendication 1 ou 2, dans lesquelles les radicaux M¹ et M² sont choisis dans le groupe constitué par H (hydrogène) et Na (sodium).

4. Utilisation des compositions selon l'une quelconque des revendications 1 à 3 pour des agents cosmétiques, ainsi que des détergents.

5. Utilisation des compositions selon la revendication 4 pour des agents cosmétiques sous la forme de shampoings pour les cheveux, de gels douche, de savons, de détergents synthétiques, de pâtes lavantes, de lotions lavantes, de préparations de brossage, de bains moussants, de bains d'huile, de bains douche, de mousses de rasage, de lotions de rasage, de crèmes de rasage et de produits pour le soin des dents.

6. Utilisation des compositions selon la revendication 4 pour des agents de faible pH pour le nettoyage de surfaces dures, tels que des détergents pour la salle de bain et les toilettes et analogues, ainsi que pour des gels nettoyants et/ou parfumés destinés à une utilisation dans des installations sanitaires.
